(19) Europäisches Patentamt
European Patent Office
Office européen des brevets

(11)  **EP 2 559 417 B1**

(12)  **EUROPEAN PATENT SPECIFICATION**

(45) Date of publication and mention
of the grant of the patent:
**19.03.2014  Bulletin 2014/12**

(51) Int Cl.:
*A61K 8/02* *(2006.01)*      *A61Q 9/04* *(2006.01)*
*A45D 26/00* *(2006.01)*

(21) Application number: **11180353.2**

(22) Date of filing: **07.09.2011**

(54) **Depilatory article with substrate**

Enthaarungsmittel mit Substrat

Article d'épilation doté d'un substrat

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2011  US 524367 P**

(43) Date of publication of application:
**20.02.2013  Bulletin 2013/08**

(73) Proprietor: **The Procter & Gamble Company
Cincinnati, OH 45202 (US)**

(72) Inventors:
• **Smith, Paul  James
  Whitton, Middlesex TW2 6EB (GB)**
• **Sagel, Paul  Albert
  Maineville, OH Ohio 45039 (US)**

• **Passi, Rajeev Kumar
  West Chester, OH Ohio 45069 (US)**
• **Mitra, Shekhar
  Cincinnati, OH Ohio 45243 (US)**
• **Broyles, Norman Scott
  Hamilton, OH Ohio 45011 (US)**

(74) Representative: **Kohol, Sonia
  Technical Centres Limited
  Procter & Gamble Patent Department
  Rusham Park
  Whitehall Lane
  Egham, Surrey TW20 9NW (GB)**

(56) References cited:
**FR-A- 1 287 305      FR-A1- 2 105 039
FR-A1- 2 626 468      US-A- 2 954 324
US-A- 3 808 637      US-A- 4 282 877
US-B1- 6 336 462**

**Description**

FIELD OF THE INVENTION

The present invention relates to depilatory articles comprising a chemically active depilatory composition disposed on a substrate.

BACKGROUND OF THE INVENTION

Depilatory compositions used to remove unwanted hair by chemical activity are known. Such compositions may comprise reducing agents to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion and provides no means of occluding the depilatory composition to prevent it from drying out. By disposing the depilatory composition on a substrate one may overcome or mitigate such disadvantages. Substrate-based depilatory products are known from JP6192056A, US2006002878, JP6135826A, JP11012123A and JP62230711A. JP63073910A in particular considers a high water content composition disposed on a substrate.

While addressing some of the usage problems of creams and lotions by removing the need for an application implement, known substrate-based depilatory compositions do not address the problem of achieving an improved removal of the article from the skin by promoting the adhesion of the depilatory composition to the substrate rather than to the skin. Depilatory compositions typically adhere to the surface of the body in order to remain in place, or to enable the depilatory article to remain in place. Upon removing the article, therefore, residual depilatory composition may remain on the surface of the body and lead to a messy and extended depilatory procedure in which the depilatory composition must be manually removed. Substrate-based articles may additionally comprise a release layer to protect the side of the depilatory composition not in contact with the substrate. Accordingly, if a release layer is being employed to protect the depilatory composition, it is important that the depilatory composition preferentially remains on the substrate rather than becoming split between the substrate and release layer. There exists a need, therefore, for a substrate-based depilatory article in which the adhesion of depilatory composition to the substrate is promoted.

SUMMARY OF THE INVENTION

According to a first aspect of the invention, the Applicants have surprisingly found that a depilatory article comprising a substrate and a depilatory composition disposed on said substrate forming a coated region of the substrate, wherein at least a portion of the surface of the coated region of the substrate has an average surface free energy measured by the method herein of at least 30 mJ/m$^2$, preferably from 31 mJ/m$^2$ to 72 mJ/m$^2$, more preferably from 32 mJ/m$^2$ to 55 mJ/m$^2$, even more preferably from 33 mJ/m$^2$ to 46 mJ/m$^2$ and even more preferably still from 34 mJ/m$^2$ to 38 mJ/m$^2$ meets the aforementioned need by encouraging the depilatory composition to adhere to the substrate rather than the arm or, in particular, a release layer removably attached to the depilatory composition.

According to a second aspect of the invention, a cosmetic method of removing hair from the skin is provided, comprising the steps of applying a depilatory article according to the first aspect of the invention to a surface of the body, preferably the human body, leaving said depilatory article in contact with the skin for a period of at least 1 minute, preferably 2 to 10 minutes, more preferably 2 to 8 minutes, removing said depilatory article from the surface of the skin, and preferably rubbing, scraping, rinsing or wiping the surface of the skin in the area to which the depilatory article was applied.

According to a third aspect of the invention, a depilatory kit is also provided, comprising: a depilatory article according to the first aspect of the invention, optionally at least one of a pre-treatment skin care composition, a post-treatment skin care composition and/or a tool to assist removal of hair and/or depilatory composition after use, and packaging for said depilatory kit.

BRIEF DESCRIPTION OF THE DRAWINGS

Fig.1. is a plan view of a depilatory article of the present invention.
Fig.2. is a side view of a depilatory article of the present invention.
Fig.3. is a side view of a depilatory article of the present invention applied to keratinous tissue.

DETAILED DESCRIPTION OF THE INVENTION

As used herein, the term "buffering base" refers to a base capable of opposing pH changes by means of chemical or physical (solubility) processes and thereby limiting the pH to less than or equal to 13.

As used herein, the term "water impermeable" includes materials or objects through which water in its liquid state does not pass.

As used herein, the term "water soluble" includes materials which have a solubility of at least 0.01 g/dm$^3$, preferably at least 0.1 g/dm$^3$, more preferably at least 0.5 g/dm$^3$, even more preferably at least 0.8 g/dm$^3$ and even more preferably still at least 1 g/dm$^3$.

As used herein the term "colloid-forming" includes chemical species that are able to form stable, aqueous solid-in-liquid colloidal systems, including nano-colloidal systems.

As used herein, the term "sodium silicate" refers to $Na_2SiO_3$, any other silicate comprising sodium as the only cation besides silicon, and any other silicate comprising sodium. The same definition applies correspondingly to any other silicate, for example "potassium silicate" refers to $K_2SiO_3$, any other silicate comprising potassium as the only cation besides silicon and any other silicate comprising potassium, "ammonium silicate" to $(NH_4)_2SiO_3$, any other silicate comprising ammonium as the only cation besides silicon and any other silicate comprising ammonium and "manganese silicate" to $Mn_2SiO_4$, any other silicate comprising manganese as the only cation besides silicon and any other silicate comprising manganese.

As used herein, all percentages are by weight of the depilatory composition, unless otherwise specified.

Depilatory articles of the present invention comprise a substrate to facilitate application of the depilatory composition to keratinous tissue and prevent a messy usage experience. The side of the substrate contacting the depilatory composition has, at least over the area which contacts the depilatory composition, an average surface free energy within the corresponding range specified below. Applicants have found that selecting a substrate with this attribute promotes adhesion of the depilatory composition to the substrate in order to reduce messiness of the depilatory procedure and enable a cleaner removal of the depilatory article from the surface of the body with less residual depilatory composition remaining on the skin.

Depilatory articles of the present invention comprise a depilatory composition in contact with a surface of the substrate, forming a coated region of the substrate. The depilatory composition may be disposed on one surface of the substrate, that surface being a depilatory surface of the substrate. The depilatory composition should be suitable for being placed in contact with a user's skin (and unwanted hair). The concentration of water in the depilatory composition is equal to or greater than 40%, preferably from 50% to 95%, more preferably from 60% to 90% and even more preferably from 70% to 90%, by weight of the depilatory composition. This high water level helps to improve the overall skin mildness of the depilatory composition by being dilute, and to keep the system more robust to pH changes, which may result in skin irritation. The depilatory article may further comprise a release layer on the opposite side of the depilatory composition to the substrate.

According to the present invention, the surface of the coated region of the substrate has an average surface free energy (SFE) of at least 30 mJ/m$^2$, preferably from 31 mJ/m$^2$ to 72 mJ/m$^2$, more preferably from 32 mJ/m$^2$ to 55 mJ/m$^2$, even more preferably from 33 mJ/m$^2$ to 46 mJ/m$^2$, even more preferably still from 34 mJ/m$^2$ to 38 mJ/m$^2$ and yet more preferably from 35 mJ/m$^2$ to 37 mJ/m$^2$. Surface free energy may readily be measured using a Sessile drop technique to determine contact angles of liquids on the surface and the well established Fowkes method to calculate the surface free energy. As used herein, the technique was followed by measuring advancing contact angles using two different liquids (water and diiodomethane, the same liquids as used in ASTM D7490-08 which describes a method of measuring surface free energy) on a Drop Shape Analyser DSA100 from Kruss, Germany. The drop volume was 3 μl. Contact angles were measured in at least fifteen different areas upon each sample tested. A summary of the Fowkes method follows.

When a droplet of liquid comes in contact with a solid substrate, it either completely wets the surface forming a thin film with no defined line of contact or it spreads to a limited extent, leaving a well defined three phase line of contact. The wettability of the liquid on the solid substrate depends not only on the SFE of the solid-liquid interface ($\gamma_{SL}$), but also on the SFE of the solid ($\gamma_S$) and on the SFE of the liquid ($\gamma_L$). Young's equation gives the relationship between these quantities:

$$\gamma_S = \gamma_{SL} + \gamma_L \cos\theta_i \qquad \text{(Equation 1)}$$

where $\theta_i$ is the contact angle between solid and the measuring liquid, *i*.

Fowkes method assumes that the SFE of a solid or liquid is a sum of independent components, associated with specific interactions

$$\gamma_s = \gamma_s^d + \gamma_s^p + \gamma_s^o \qquad \text{(Equation 2)}$$

Where ($\gamma^d_S$) and ($\gamma^p_S$) are the dispersion and polar components respectively, and ($\gamma^o_S$) refers to all remaining interactions, which are neglected according to the method as used herein. A corresponding equation applies for $\gamma_L$. For two-phase systems containing a solid and liquid, the SFE corresponding to the solid-liquid interface is estimated by

$$\gamma_{SL} = \gamma_S + \gamma_L - 2(\gamma_S^d \gamma_L^d)^{0.5} - 2(\gamma_S^p \gamma_L^p)^{0.5} \qquad \text{(Equation 3)}$$

Using a liquid (1) with essentially only a dispersion interaction, i.e. $\gamma^p_{L1} = 0$ (diiodomethane according to ASTM D7490-08), then equations 1 and 3 may be combined to reach

$$\gamma_S = \gamma_S^d = \gamma_{L1}^d (1 + \cos\theta_1)^2 / 4 \qquad \text{(Equation 4)}$$

Where $\gamma_{L1} = \gamma^d_{L1}$

Equation 4 allows the determination of $\gamma^d_S$ which is followed by measuring the contact angle for a liquid (2) with a polar interaction, i.e. $\gamma^p_{L2} \neq 0$, hence $\gamma_{L2} = \gamma^d_{L2} + \gamma^p_{L2}$ *(water according to ASTMD7490-08)* and $\gamma^p_S$ is calculated by

$$\gamma_S^p = \{0.5\gamma_{L2}(1 + \cos\theta_2) - (\gamma_S^d \gamma_{L2}^d)^{0.5}\}^2 / \gamma_{L2}^p \qquad \text{(Equation 5)}$$

The total SFE of the substrate is the sum of the dispersion and polar components for the solid:

$$\gamma_S = \gamma_S^d + \gamma_S^p$$

As used herein, water and diiodomethane have been used as measuring liquids. Diiodomethane is nonpolar with $\gamma_{L1} = \gamma^d_{L1} = 50.8$ mJ/m$^2$. Water, conversely, has a dominant polar component, $\gamma^p_{L2} = 51.0$ mJ/m$^2$ and $\gamma^d_{L2} = 21.8$ mJ/m$^2$.

Further details of the Fowkes method may be found in the following publications:

Fowkes, F.M., ATTRACTIVE FORCES AT INTERFACES. Industrial & Engineering Chemistry, 1964. 56(12): p. 40-52.
Fowkes, F.M., Donor-Acceptor Interactions at Interfaces. The Journal of Adhesion, 1972. 4(2): p. 155 - 159.

In a desired embodiment, the individual measurements of the surface free energy should not vary greatly between measurement points. Accordingly, it is preferred that the range of the measurements (i.e. the lowest individual measurement for a specific sample subtracted from the largest individual measurement) of the surface free energy made according to the method above is less than 10 mJ/m$^2$, preferably less than 5 mJ/m$^2$, more preferably less than 3 mJ/m$^2$, even more preferably less than 2 mJ/m$^2$ and even more preferably still less than 1 mJ/m$^2$.

The surface free energy of a substrate or release layer surface may be increased by any known means prior to applying the depilatory composition, for example corona treatment which uses an electric discharge to ionize surface molecules, atmospheric plasma treatment, flame plasma treatment, chemical plasma treatment, topical surfactant application (by, for example functionalised silicones, cationic quaternary ammonium compounds, anionic phosphates or sulphates, and nonionic esters, alcohols and ethoxylates), or the incorporation of surfactants within the substrate (by, for example the topical surfactants above added into a hot-melt of a polymer resin). Preferably, corona treatment, atmospheric plasma treatment, flame plasma treatment or chemical plasma treatment is used and more preferably corona treatment is used.

The surface free energy of a substrate or release layer surface may be decreased using topical treatments, for example fluoropolymers, non-functionalised silicones (e.g. polydimethylsiloxane (PDMS)) and fluorosilicones.

Advantageously, at least a portion of the surface of the coated region of the substrate has a roughness at a magnification of x50 of at least 0.08 $\mu$m, preferably 0.09 $\mu$m, more preferably 0.1 $\mu$m, even more preferably 0.11 $\mu$m

and even more preferably still 0.12 μm. It is also, and further, preferred that the surface of the contacting region of the release layer has a roughness at a magnification of x50 of less than 0.12 μm, preferably less than 0.11 μm, more preferably less than 0.1 μm and even more preferably less than 0.09 μm. Notably, it is a preferred embodiment if the surface of the contacting region of the release layer has a roughness less than that of the surface of the coated region of the substrate.

Surface roughness may readily be measured using a LEXT OLS3100 (Version 6.0 software) Laser Scanning Confocal Microscopy operating at x50 magnification. Both optical and confocal images are taken. Roughness measurements may be made on the confocal images on a magnification of x50. After image acquisition the automatic 'Spike Removal' and 'Surface tilt Correction' procedures are carried out by the software and the total area surface roughness calculated using the software. Non-representative defects (scratches etc) are avoided in selecting the area of interest for measurement.

Standard deviations of the roughness values are calculated using three measured roughness values of three areas from each sample. For each of the measurements identical conditions are used so the values are comparable and a bar diagram of the measured roughness along with standard deviations of all the samples may be plotted.

Surface roughness is normally an inherent property of a commercially available substrate or release layer material, but may be decreased by passing the material over/through non-textured rollers or nip rollers during the manufacturing process. Conversely to increase surface roughness, a material may be passed over/through textured rollers and/or embossment calendars or sand blasted during or after the manufacturing process.

In another advantageous embodiment, the surface free energy and/or roughness is within one of the ranges above over at least 40% of the surface area of the coated region, preferably over at least 70% of the surface area of the coated region, more preferably over at least 90% of the surface area of the coated region and even more preferably over the whole of the coated region, in order to further promote the effective release of the depilatory article from the surface of the body and reduce the amount of any residue.

As previously mentioned, depilatory articles of the present invention may comprise a protective release layer removably attached to the depilatory composition, forming a contacting region of the release layer, preferably on a surface of the depilatory composition substantially opposing that which is in contact with the substrate. Advantageously, irrespective of the average surface free energy of the coated region of the substrate, the surface of the contacting region of the release layer has an average surface free energy less than the average surface free energy of the coated region of the substrate, preferably the difference is at least 2mJ/m$^2$, more preferably the difference is at least 4 mJ/m$^2$, even more preferably the difference is at least 6 mJ/m$^2$, even more preferably still the difference is at least 8 mJ/m$^2$ and yet more preferably the difference is at least 10 mJ/m$^2$. For example, the contacting region of the release layer may have an average surface free energy of less than 35 mJ/m$^2$, preferably less than 32 mJ/m$^2$, more preferably less than 30 mJ/m$^2$ and even more preferably less than 28 mJ/m$^2$.

The protective release layer may comprise materials including polymer resins such as a polyolefin e.g. polypropylene (including stratified biaxially oriented polypropylene (SBOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers, mixtures or combinations of said materials. Where appropriate the protective relase layer may comprise one or more laminations, combinations of multiple layers and/or indications (which may include instructions and illustrations) relating to at least one aspect of the usage of the depilatory article. In an advantageous embodiment the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated region of the substrate to provide a removal tab.

The rheological properties of the depilatory composition may also lead to improved performance in use. In particular, the yield point describes the resistance of the depilatory composition to deformation under environmental stress. If the yield point is too high, then the depilatory composition may not deform sufficiently, with hair fibres unable to enter the depilatory composition effectively upon application, resulting in less desirable depilatory effectiveness. If the yield point is too low, however, then the depilatory composition may flow during storage, transport or use and is not cleanly removed from the skin upon removal of the depilatory article, thus requiring the inconvenience of additional wiping and risking irritation to the user. A poor yield point is especially undesirable when a protective release layer is used as the composition may break apart as the substrate and release layer to which it adheres are pulled apart. Accordingly, the depilatory composition preferably has a yield point from 25 Pa to 2000 Pa, more preferably from 40 Pa to 1000 Pa, even more preferably from 55 Pa to 500 Pa and even more preferably still from 70 Pa to 250 Pa, when measured via a stress controlled amplitude sweep at a frequency of 1 Hz and a temperature of 25°C. The yield point described is defined herein as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

Advantageously, the depilatory composition displays an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the depilatory composition exceeds its viscous modulus at a low frequency of applied stress. This indicates that the depilatory composition is behaving in a solid-like manner at rest and is of particular benefit when the depilatory composition is interposed between two substrates, for example a substrate and a protective release layer.

In another preferred embodiment, the depilatory composition displays a high degree of shear thinning behaviour enabling the effective coating of target hairs during application and improve depilatory efficacy. Accordingly, at a low shear rate of $0.1 \text{ s}^{-1}$ the dynamic viscosity of the depilatory composition is preferably 1000 Pa.s to 10000 Pa.s measured at a temperature of 25°C, whereas at a high shear rate of $1000 \text{ s}^{-1}$, the dynamic viscosity of the depilatory composition is preferably 0.1 Pa.s to 1 Pa.s, measured at a temperature of 25°C.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. It may be desirable to utilize gel network structures or oil-in-water emulsions to thicken the depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01% to about 20%, preferably from about 0.1% to about 10%, more preferably from about 0.3% to about 5%, and even more preferably from about 0.5% to about 4%, by weight of the depilatory composition.

Advantageously, the thickening agent comprises carrageenan. The carrageenan is preferably present in an amount of from 0.1 % to 10%, more preferably from 0.5% to 8%, even more preferably from 1% to 5% and even more preferably still from 2% to 4% by weight of the depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

Preferably, the depilatory composition is disposed upon the substrate in an amount per unit area of $0.300 \text{ g/cm}^2$ to $0.001 \text{ g/cm}^2$, more preferably from $0.015 \text{ g/cm}^2$ to $0.003 \text{ g/cm}^2$, even more preferably from $0.080 \text{ g/cm}^2$ to $0.005 \text{ g/cm}^2$ and even more preferably still from $0.05 \text{ g/cm}^2$ to $0.005 \text{ g/cm}^2$, wherein the unit area refers to the coated region of the substrate and not including any uncoated surface of the substrate. Within these preferred ranges, sufficient depilatory composition is provided to coat the hairs to improve depilatory activity, but little enough to discourage rupturing of the depilatory composition when any protective release layer is removed from the depilatory article, or the depilatory article is removed from the surface of the body. Additionally, the area used to calculate the amount of depilatory composition disposed upon the substrate is calculated ignoring any surface texturing or micro-structuring. Alternatively, the mean thickness of the depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

Advantageously, the substrate possesses a rigidity in the range of from 5.00 g/cm to 0.05 g/cm, preferably from 3.00 g/cm to 0.05 g/cm and more preferably from 1.80 g/cm to 0.08 g/cm, even more preferably from 0.80 g/cm to 0.10 g/cm and even more preferably still from 0.60 g/cm to 0.20 g/cm. This rigidity of the substrate promotes desirable handleability and conformability attributes of the depilatory article. In particular, the article collapsing under gravity or folding is avoided, which is especially undesirable if different areas of the depilatory composition are able to readily come into contact with each other, while maintaining the capability for the depilatory article to conform to the surface to which it is applied without folding or crinkling, in order to further improve depilatory efficiency. Accordingly, the substrate is readily conformable to the skin and unwanted hair without permanently deforming during use, as this may also result in problems for the user during application. In a preferred embodiment, the rigidity is substantially constant and does not change during the lifetime of a product.

Rigidity can be readily measured using the American Standard Test Method (ASTM) D2923-06, method B (i.e. using a powder to reduce the effect of static electricity) on a Handle-O-Meter, model #211-300, available from Thwing-Albert Instrument Co. of Philadelphia, Pa. The rigidity is expressed as grams per centimetre of sample width. Samples were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a substrate is a function of substrate thickness and inherent modulus of elasticity. Different materials have different moduli of elasticity. Based upon the material or materials that the substrate comprises, a substrate thickness should be selected that enables the desired rigidity of the substrate to be achieved.

The substrate may be water permeable or water impermeable. The substrate may comprise any suitable material such as fibrous materials, papers, fabrics, non-wovens, plastics, amorphous solids, crystalline solids, foils, rubbers, latex, thermoplastic elastomers, cellular foams (open and closed cell), composites, laminates and mixtures thereof. Preferably, the substrate is water impermeable. Using a water impermeable substrate prevents water loss from the depilatory composition while the depilatory composition is in contact with the keratinous tissue and thus prevents the depilatory composition from drying out. Water loss from the depilatory composition lowers the water concentration, thus increasing the concentration of active ingredients and bases present. This could result in irritation to the skin, which applicants wish to avoid, and may alter the nature of the composition to the detriment of adhesion to the substrate surface.

The substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The substrate may comprise a single polymer or mixtures of polymers or copolymers. Preferably the substrate comprises a plastic sheet, more preferably a polyolefm, even more preferably a polyethylene and even more preferably still high density polyethylene.

In an advantageous embodiment, the depilatory composition is disposed upon the water impermeable material, preferably plastic sheet, more preferably polyolefin, even more preferably polyethylene and even more preferably still high density polyethylene. In this advantageous embodiment, there is preferably no layer of water permeable material between the depilatory composition and the water impermeable material. In a preferred embodiment, the water impermeable material forms a water impermeable layer.

The substrate preferably has a thickness of from 80 $\mu$m to 12 $\mu$m, more preferably from 50 $\mu$m to 15 $\mu$m, even more preferably from 40 $\mu$m to 16 $\mu$m, and even more preferably still from 30 $\mu$m to 17 $\mu$m.

The substrate may be a laminate comprising at least two materials, including non-wovens; paper; board; metal based substrates (eg aluminium foil); flocking or topical coatings (e.g. surfactants; printing); closed or open cell foams or substrates described herein above. In a preferred embodiment, at least one of the materials is water impermeable.

The substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

A layer of depilatory composition can be applied to the substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of depilatory composition disposed on the substrate. For example, the speed at which the substrate travels through an extrusion process determines the quantity of depilatory composition disposed upon said substrate. The area of depilatory composition may cover the entire surface of the substrate of a portion thereof. Advantageously, the depilatory composition covers less than the entire surface of the substrate to facilitate handling. The substrate may comprise at least one region with two orthogonal dimensions each of a length greater than 1 cm, preferably greater than 1.5 cm and more preferably greater than 2 cm upon which no depilatory composition is disposed.

In a preferred embodiment, the depilatory composition comprises a keratin reducing agent to weaken and/or break strands of unwanted hair. Non-limiting examples of suitable keratin reducing agents include: sulphide salts such as $Li_2S$, $Na_2S$, $K_2S$, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof. In a preferred embodiment, the concentration of the conjugate acid of the thioglycolate salt, (which includes all species in the acid's deprotonation equilibrium system) is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the depilatory composition. Advantageously, the quantity of monovalent cations (or monovalent metal cations in the preferred embodiment above) per unit area of the aforementioned coated region is less than $5.10 \times 10^4$ mol/cm$^2$, preferably less than $3 \times 10^{-4}$ mol/cm$^{-2}$, more preferably from $1 \times 10^{-9}$ mol/cm$^2$ to $1.5 \times 10^{-4}$ mol/cm$^2$, even more preferably from $2.50 \times 10^{-8}$ mol/cm$^2$ to $6.65 \times 10^{-5}$ mol/cm$^2$ and even more preferably still from $6 \times 10^{-7}$ mol/cm$^2$ to $4.5 \times 10^{-5}$ mol/cm$^2$.

The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula if monovalent ion containing thioglycolate salts or bases are used. Accordingly, in an advantageous embodiment, the depilatory composition comprises a divalent cation" preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

In a depilatory composition comprising a mixture of monovalent and divalent ions, controlling the ratio of divalent ions to monovalent ions may also improve the safety characteristics of the depilatory articles of the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the depilatory composition. Accordingly, in an alternative embodiment the ratio of the concentration of divalent ions to the concentration of monovalent ions present in the depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 to 1:1.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide.

In a preferred embodiment, the base is present at a concentration of from 0.1 % to 10.0%, more preferably from 0.5% to 8.0% and even more preferably from 1.0% to 5.0%, by weight of the depilatory composition.

In another preferred embodiment, the depilatory composition comprises at least one silicate or silica, advantageously at least one water-soluble or colloid-forming silicate or silica, in order to enhance depilatory effectiveness.

Preferably, the depilatory composition comprises at least one water-soluble or colloid-forming silicate selected from lithium silicates; sodium silicates (including disodium metasilicate pentahydrate and disodium metasilicate nanohydrate); potassium silicates; calcium silicates, ammonium silicates; manganese silicates; imidazolium silicates, synthetic and natural silicates (clays) or mixtures thereof. More preferably, the depilatory composition comprises at least one water-soluble or colloid-forming silicate selected from synthetic clays; sodium silicates, potassium silicates, or mixtures thereof and even more preferably the depilatory composition comprises a sodium silicate or mixtures of sodium silicates.

Alternatively, the depilatory composition comprises a form of silica that is colloid-forming, (such as amorphous microporous silica), forms sol or gel systems, (such as silica gels and nano-colloidal silicas), or is mesostructured. Surface modification of silica may be advantageous to promote the formation of stable colloid systems.

Suitable synthetic and natural silicates (clays) are available commercially as: Laponite® RDS; XLS and S etc. (available from RockWood Additives Limited); Wyoming Bentonite; Californian Hectorite; Jadeite; Enstaite and Rhodonite; Benonate® EW (available from Rheox Inc.); Bentolite® (available from Southern Clay Products Inc.) Optigel® (available from Süd Chemie Rheologicals)

The silicate or silica is preferably present in the depilatory composition in an amount per unit area of the coated region of from $2.05 \times 10^{-b}$ mol/cm$^2$ to $1.23 \times 10^{-4}$ mol/cm$^2$, preferably from $1.64 \times 10^{-7}$ mol/cm$^2$ to $3.69 \times 10^{-5}$ mol/cm$^2$ and more preferably from $4.92 \times 10^{-7}$ mol/cm$^2$ to $8.20 \times 10^{-6}$ mol/cm$^2$. Within the preferred ranges, the effectiveness of the depilatory composition is further increased while irritation is maintained within an acceptable level. Without wishing to be bound by theory, applicants believe that an amount of silicate or silica is required in order to enhance the dissociation of the thioglycolate salt sufficiently for the increase in efficacy to be clearly apparent to the user, but that excessive dosage of silicate or silica may lead to over-dissociation of the thioglycolate salt resulting in increased skin irritation. Alternatively, the silicate or silica may be present in the depilatory composition in an amount of from 0.01% to 5%, preferably 0.1% to 4%, more preferably 0.2% to 3% and even more preferably from 0.5% to 2% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in hair removal compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Depilatory articles of the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the substrate of the depilatory article is substantially planar.

The coated region preferably comprises an upper-lip portion adapted to be placed above a human mouth, and a first return portion projecting from the upper lip portion and adapted to be placed contiguously with the outer extremity of the vermilion lip in a first corner of the mouth. The return portion has a length along its greatest dimension of at least 0.2 cm, preferably from 0.5 cm to 5 cm, more preferably from 0.75 cm to 4 cm, even more preferably from 1 cm to 3 cm. Applicants have found that this configuration enables the user to remove unwanted hair from the skin immediately surrounding the corner of the mouth while lowering the risk of depilatory composition contacting the vermillion lip, where it may cause irritation. In an alternative embodiment, the coated region further comprises a second return portion projecting from the upper lip portion and adapted to be placed contiguously with the outer extremity of the vermillion lip in a second corner of the mouth.

Advantageously, the upper lip portion has a length along its greatest dimension of at least 0.2 cm, preferably from 0.5 cm to 15 cm, more preferably from 1 cm to 12 cm, even more preferably from 2 cm to 10 cm and even more preferably still from 3 cm to 8 cm. This dimension enables the upper lip portion to cover a desirable length of the upper lip and thus achieve the desired depilatory action. In a preferred embodiment, the upper lip portion is adapted to be placed to be at least partially contiguously with the upper border of the upper vermilion lip, to enable depilatory action to be achieved on the skin immediately surrounding the upper vermilion lip while lowering the risk of depilatory composition contacting the upper vermilion lip, where it may cause irritation.

In another preferred embodiment, the coated region comprises a lower lip portion adapted to be placed below a human mouth, preferably wherein the lower lip portion is adapted to be placed to be least partially contiguously with the lower border of the lower vermilion lip to enable depilatory action to be achieved on the skin immediately surrounding the lower vermilion lip while lowering the risk of depilatory composition contacting the lower vermilion lip, where it may cause irritation.

Depilatory articles of the present invention may comprise at least one finger-tab being substantially free of depilatory composition to enable handling of the depilatory article. Two or more finger-tabs may be positioned on substantially opposing sides of the coated region. These finger tabs enable a user to apply tension to the coated region of the substrate. Surprisingly, applicants have found that applying tension across the coated region of the depilatory article creates an effect of temporarily causing the coated region to exhibit an apparent increased rigidity, enabling the user to accurately position the coated region, and hence depilatory composition on to the desired region of the body. Tensioning the coated region may be achieved in a number of ways, non-limiting examples of which include holding the depilatory article either side of the coated region, for example with the hands or a tool, so as to apply tension between the areas being held. Alternatively, depilatory articles of the present invention may comprise at least one finger-tab being substantially free of depilatory composition and positioned to allow the weight of the article to tension the coated region when being held by the finger-tab.

In a preferred embodiment, at least one finger tab extends from the perimeter of the coated region by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated region by a minimum of 1 cm, preferably from 1.5 cm to 5 cm, more preferably from 2 cm to 4 cm and even more preferably from 2.5 cm to 3.5 cm, in order to aid handling of the depilatory article.

In a preferred embodiment, the depilatory articles of the present invention are packaged to prevent water loss and/or oxygen permeation. Alternatively, the depilatory articles of the present invention are packaged in water impermeable packaging. Examples of suitable packaging materials include films of EVOH; PP; PE; Nylon; foil laminates (including metalized PET; BOPP and PE), mixtures thereof, laminates thereof or multi-laminates thereof. More preferably, the packaging comprises an inert gas and even more preferably the inert gas comprises at least one of nitrogen, argon or carbon dioxide. Alternatively, the packaging comprises a partial vacuum.

A second aspect being a method of removing hair from the skin is also provided by the present invention, comprising the steps of:

> (a) applying a depilatory article according to the present invention to the surface of the skin, preferably mammalian and more preferably human skin,
> (b) leaving said depilatory article in contact with the skin for a period of at least 1 minute, preferably 2 to 10 minutes, more preferably 2 to 8 minutes,
> (c) removing said depilatory article from the surface of the skin, and
> (d) preferably rubbing, scraping, rinsing or wiping the surface of the skin in the area to which the depilatory article was applied.

Advantageously, the method of removing hair from the skin further comprises the step of tensioning the coated region of the depilatory article prior to applying it to the skin.

The same means used to apply tension to the coated region may be used to ensure that the depilatory article is applied to the surface of the body such that the coated region is applied under tension to the unwanted hair in order to maintain the improved handling characteristics described above. In a preferred embodiment, the tension is kept substantially constant during application of the depilatory article. The flexible nature of the substrate allows the substrate to conform to the surface of the body to offer improved contact between the depilatory composition and the unwanted hair. In a preferred embodiment, the tension may be at least partially, more preferably substantially completely released from the coated region after applying the depilatory article to the skin in order to improve the conformability of the depilatory article.

A third aspect being a depilatory kit is also provided by the present invention, which comprises at least one depilatory article of the present invention, packaging for said depilatory article(s), and optionally at least one of a third component selected from:

a) a pre-treatment skin care composition which may comprise ingredients to promote skin conditioning (e.g. emollients), hair hydration or provide a skin barrier (e.g. hydrophobic materials) and intended for use prior to applying the depilatory article.

b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.

c) a tool to assist in the removal of hair and/or depilatory composition from the skin.

d) indications (which may include instructions and/or illustrations) relating to at least one aspect of usage of the depilatory article or another component of the kit.

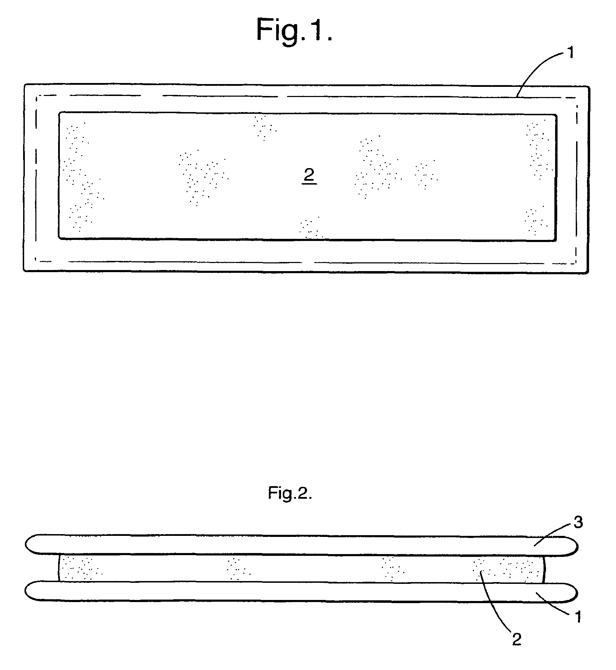

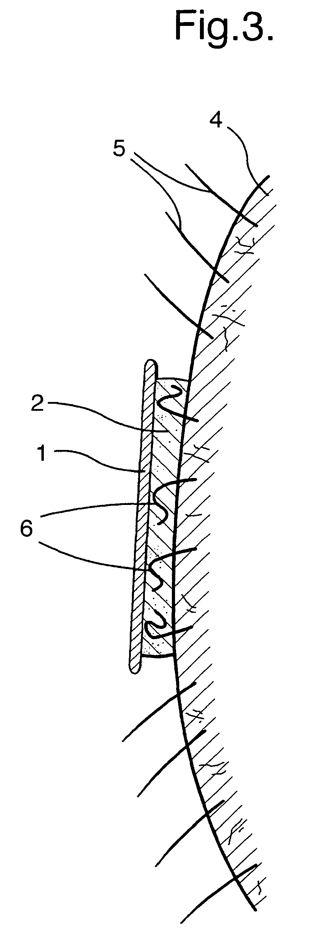

Reference is made to the figures, which disclose a non-limiting embodiment of the invention. Fig.1 depicts a plan view of a depilatory article of the present invention, comprising a substrate (1) and a depilatory composition (2). Fig. 2 depicts a side view of a depilatory article of the present invention, further comprising a protective release layer (3). Fig.3 depicts a side view of a depilatory article of the present invention in use, i.e. applied to keratinous tissue which comprises the skin (4), hair strands (5) outside the depilatory composition(2) and hair strands (6) within the depilatory composition(2).

Example

The following examples further describe and demonstrate one embodiment within the scope of the present invention. The examples are given solely for the purpose of illustration and are not to be construed as a limitation of the present invention, as many variations thereof are possible.

| Formulation Ingredients | % w/w |
|---|---|
| DI water | 87.05 |
| Carrageenan CI-123[1] | 2.60 |
| Sodium Silicate Solution (42% w/w in water)[2] | 1.60 |
| Calcium Hydroxide[3] | 3.25 |
| Calcium Thioglycolate Trihydrate[4] | 5.50 |
| 1 Carrageenan CI-123 available from CPKelco<br>2 Sodium Silicate Solution (42% w/w in water) available from Cognis<br>3 Calcium Hydroxide Reag. Ph. Eur. puriss. p.a. available from Sigma-Aldrich Co.<br>4 Calcium Thioglycolate Trihydrate 99.8% available from BRUNO BOCK Chemische Fabrik GmbH & Co. | |

A 400 ml speed mixer plastic pot was sanitized and DI water weighed in directly. The calcium hydroxide was added with mixing followed by the slow addition of the sodium silicate solution with mixing. The Carrageenan was then slowly added to the batch with mixing to ensure hydration, adjusting mixing speeds and time as required. The Calcium Thioglycolate was then added with mixing, again adjusting mixing speeds and time as required. The batch was then milled using an IKA T50 (5,200) rpm for 2 minutes. The pH was measured and the batch stored in glass pots with no head space.

| | Comparative Example | Inventive Example A | Inventive Example B | Inventive Example C | Inventive Example D | Comparative Example E |
|---|---|---|---|---|---|---|
| **Substrate** | BOPP Non-fluoropolymer side[2] | BOPP Corona treated side[3] | BOPP Corona treated side[3] | OPP Corona treated side (UHSE)[4] | Melinx S PET[5] | HDPE/LDPE blend[6] |
| Surface free energy $[mJ/m^2]$ | 27.80 | 36.50 | 36.10 | 35.60 | 46.90 | 30.7 |
| Roughness $[\mu m]$ | 0.100 | 0.096 | 0.076 | 0.063 | 0.035 | 2.14 |
| **Release layer** | BOPP Fluoropolymer surface[1] | BOPP Fluoropolymer surface[1] | BOPP Non-fluoropolymer surface[2] | BOPP Non-fluoropolymer surface[2] | BOPP Non-fluoropolymer surface[2] | BOPP Non-fluoropolymer surface |
| Surface free energy $[mJ/m^2]$ | 23.40 | 23.40 | 27.80 | 27.80 | 27.80 | 27.80 |
| Roughness $[\mu m]$ | 0.085 | 0.085 | 0.100 | 0.100 | 0.100 | 0.100 |
| $\Delta$surface free energy $[mJ/m^2]$ | 4.4 | 13.10 | 8.70 | 7.8 | 19.1 | 19.1 |
| Release success[7] / Total tested | 0/5 | 5/5 | 55 | 5/5 | 5/5 | 5/5 |

1 and 2 Available from 3M Company under the tradename 3M Scotchpak™ 9741 (fluoropolymer coated on one side [23.40 $mJ/m^2$] and non-fluoropolymer treated on opposite side [27.80 $mJ/m^2$]).
3 Available from Innovia under tradename Rayoart CG90.
4 Available from Toray Industries under the tradename F62W 19 microns UHSE side.
5 Available from HiFi as Melinex S 19 microns.
6 HDPE/LDPE film (LBI 85% M6030 and Exxon Mobil 15% LD2001) manufactured on a Merritt-Davis casting line (thickness 23 microns).
7 Release success is the number of strips where the depilatory composition cleanly releases from the release layer such that < 0.5 $cm^2$ remains on the release layer (as measured by placing the release layer on $5 \times 5$ mm square graph paper such that the release layer is flat and horizontal in direct contact with the graph paper and the squares counted where the depilatory composition residue is present).

The above depilatory composition was disposed to a thickness of 0.3 mm, width of 1.5 cm and length of 3.5 cm onto the substrate in the comparative example (substrate 4.6 cm in length and 1.7 cm in width) using a stencil and wiper blade, such that the area covered by the depilatory composition was centered along the width of the film and 1 mm away from the perimeter edge of one end of the film's length, thus providing a 1 mm overhang of substrate at the edges of the depilatory composition along the length and at one edge across its width. The other edge across the width of depilatory composition therefore had an overhang of 10 mm. The release layer (of the same shape as the substrate) was then carefully positioned on the depilatory composition such that its edges were above the edges of the substrate.

The article was then placed such that it was horizontal on a flat lab bench with the release layer in direct contact with the lab bench. After 1.5 hours the 10 mm overhang of the release layer was held down on the flat lab bench whilst the 10 mm overhang of the substrate was grasped between finger and thumb and slowly pulled back along the length of the substrate.

The amount of depilatory composition remaining on the release layer was visually assessed. A depilatory composition residue covering an area $\geq$ 0.5 $cm^2$ remaining on the release layer was considered a release failure (as measured by placing the release layer on $5 \times 5$ mm square graph paper such that the release layer is flat and horizontal in direct contact to the graph paper and the squares counted where the depilatory composition residue is present). This was repeated 5 times and reported as the number of passes out of 5. The procedure was then repeated for each of the inventive examples.

Accordingly, it may be seen from the experiments above that selecting the substrates with the higher average surface free energy (at least 30 mJ/m$^2$) in contact with the depilatory composition improves the adhesion of the depilatory composition to the substrate and hence the release profile.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A depilatory article comprising:

   i) a substrate comprising a polyolefin; and
   ii) a depilatory composition disposed on said substrate, forming a coated region of the substrate;

   wherein at least a portion of the surface of the coated region of the substrate has an average surface free energy measured using a Sessile drop technique and Fowkes method as described herein from 31 mJ/m$^2$ to 72 mJ/m$^2$, preferably from 32 mJ/m$^2$ to 55 mJ/m$^2$, more preferably from 33 mJ/m$^2$ to 46 mJ/m$^2$ even more preferably still from 34 mJ/m$^2$ to 38 mJ/m$^2$ and yet more preferably from 35 mJ/m$^2$ to 37 mJ/m$^2$.

2. A depilatory article according to claim 1, wherein a protective release layer (3) is removably attached to the depilatory composition of the depilatory article, forming a contacting region of the release layer;
   wherein preferably the surface of the contacting region of the release layer has an average surface free energy less than the average surface free energy of the coated region of the substrate, more preferably the difference is at least 2 mJ/m$^2$, even more preferably the difference is at least 4 mJ/m$^2$, even more preferably still the difference is at least 6 mJ/m$^2$, yet more preferably the difference is at least 8 mJ/m$^2$ and even further preferably the difference is at least 10 mJ/m$^2$.

3. A depilatory article according to either preceding claim, wherein the surface of the coated region of the substrate has a roughness as measured using LEXT OLS3100 (Version 6.0) software Laser Scanning Confocal Microscope operating at x50 magnification by the method described herein at a magnification of x50 of at least 0.08 $\mu$m, preferably 0.09 $\mu$m, more preferably 0.1 $\mu$m, even more preferably 0.11 $\mu$m and even more preferably still 0.12 $\mu$m

4. A depilatory article according to claim 2 or 3 wherein the surface of the contacting region of the release layer has a roughness as measured using LEXT OLS3100 (Version 6.0) software Laser Scanning Confocal Microscope operating at x50 magnification by the method described herein at a magnification of 50x of less than 0.12 $\mu$m, preferably less than 0.11 $\mu$m, more preferably less than 0.1 $\mu$m and even more preferably less than 0.09 $\mu$m.

5. A depilatory article according to any preceding claim, wherein the depilatory composition has a yield point from 25 to 2000 Pa, preferably from 40 to 1000 Pa, more preferably from 55 to 500 Pa and even more preferably from 70 to 250 Pa measured via a stress controlled amplitude sweep at a frequency of 1Hz and a temperature of 25°C.

6. A depilatory article according to any preceding claim, wherein the depilatory composition is aqueous, preferably wherein the depilatory composition comprises water in an amount of at least 40%, preferably from 50% to 98%, more preferably from 60% to 95% and even more preferably from 70% to 90%, by weight of the aqueous depilatory composition.

7. A depilatory article according to any preceding claim, wherein the at least a portion of the coated region of the substrate has been treated by corona treatment, atmospheric plasma treatment, flame plasma treatment, chemical plasma treatment or combinations thereof, preferably wherein at least a portion of the coated region of the substrate has been treated by corona treatment.

8. A depilatory article according to any preceding claim, wherein said substrate comprises a polyethylene, preferably a high density polyethylene.

9. A depilatory article according to any preceding claim, wherein said depilatory composition is disposed on said substrate in an amount per unit area of the coated region of from 0.3 $g/cm^2$ to 0.001 $g/cm^2$, preferably from 0.015 $g/cm^2$ to 0.003 $g/cm^2$, more preferably 0.08 $g/cm^2$ to 0.005 $g/cm^2$ and even more preferably from 0.05 $g/cm^2$ to 0.005 $g/cm^2$.

10. A depilatory article according to any preceding claim, wherein said substrate has a thickness of from 80 $\mu$m to 12 $\mu$m, preferably from 50 $\mu$m to 15 $\mu$m, more preferably from 40 $\mu$m to 16 $\mu$m, and even more preferably from 30 $\mu$m to 17 $\mu$m.

11. A depilatory article according to either preceding claim, wherein the depilatory composition comprises a keratin reducing agent, preferably thioglycolic acid or a thioglycolate salt, more preferably a thioglycolate salt, even more preferably a thioglycolate salt comprising a divalent cation, even more preferably still a thioglycolate salt selected from sodium, potassium, magnesium, calcium, beryllium, strontium, zinc, monoethanolamine, ammonium, tetralkylammonium, imidazolium, pyridinium, phosphonium or glyceryl thioglycolate salts and yet more preferably calcium thioglycolate.

12. A depilatory article according to any preceding claim, wherein the depilatory composition comprises a base, preferably in a concentration range of from 0.1% to 10.0%, more preferably from 0.5% to 8% and even more preferably from 1% to 5% by weight of the depilatory composition.

13. A depilatory article according to any preceding claim, wherein the substrate is water impermeable.

14. A method of removing hair from the skin, comprising the steps of:

(a) applying a depilatory article according to any preceding claim to a surface of skin, preferably human skin,
(b) leaving said depilatory article in contact with the skin for a period of greater than 1 minute, preferably 2 to 10 minutes, more preferably 2 to 8 minutes
(c) removing said depilatory article from the surface of the skin, and
(d) preferably rubbing, scraping, rinsing or wiping the surface of the skin in the area to which the depilatory article was applied.

15. A depilatory kit, comprising:

(a) a depilatory article according to any of claims 1-13,
(b) optionally, at least one of a pre-treatment skin care composition, a post-treatment skin care composition and/or a tool to assist removal of hair and/or depilatory composition after use, and
(c) Packaging for said depilatory kit.


**Patentansprüche**

1. Depilierartikel, der Folgendes umfasst:

I) ein Substrat, das ein Polyolefin umfasst, und
II) eine Depilierzusammensetzung, die auf dem Substrat angeordnet ist und einen beschichteten Bereich des Substrats bildet,

wobei mindestens ein Teil der Oberfläche des beschichteten Bereichs des Substrats eine durchschnittliche freie Oberflächenenergie aufweist, die bei Messung mit einem Verfahren des ruhenden Tropfens (Sessile Drop) und der Methode nach Fowkes, die hierin beschrieben sind, von 31 $mJ/m^2$ bis 72 $mJ/m^2$, vorzugsweise von 32 $mJ/m^2$ bis 55 $mJ/m^2$, stärker bevorzugt von 33 $mJ/m^2$ bis 46 $mJ/m^2$, noch stärker bevorzugt jedoch von 34 $mJ/m^2$ bis 38 $mJ/m^2$ und sogar noch stärker bevorzugt von 35 $mJ/m^2$ bis 37 $mJ/m^2$ beträgt.

2. Depilierartikel nach Anspruch 1, bei dem eine Schutztrennschicht (3) lösbar mit der Depilierzusammensetzung des Depilierartikels verbunden ist und auf der Trennschicht einen Kontaktbereich bildet, wobei vorzugsweise die Oberfläche des Kontaktbereichs der Trennschicht eine durchschnittliche freie Oberflächenenergie aufweist, die kleiner als die durchschnittliche freie Oberflächenenergie des beschichteten Bereichs des Substrats ist, der Unterschied stärker bevorzugt mindestens 2 mJ/m$^2$, der Unterschied noch stärker bevorzugt mindestens 4 mJ/m$^2$, der Unterschied noch stärker bevorzugt jedoch mindestens 6 mJ/m$^2$, der Unterschied noch stärker bevorzugt mindestens 8 mJ/m$^2$ und der Unterschied zudem vorzugsweise mindestens 10 mJ/m$^2$ beträgt.

3. Depilierartikel nach einem der vorhergehenden Ansprüche, wobei die Oberfläche des beschichteten Bereichs des Substrats eine Rauheit aufweist, die bei Messung mit einem auf 50fache Vergrößerung eingestellten konfokalen Lasermikroskops LEXT OLS3100 (Softwareversion 6.0) mit der hierin beschriebenen Methode bei einer Vergrößerung von 50x mindestens 0,08 $\mu$m, vorzugsweise 0,09 $\mu$m, stärker bevorzugt 0,1 $\mu$m, noch stärker bevorzugt 0,11 $\mu$m und noch stärker bevorzugt 0,12 $\mu$m beträgt.

4. Depilierartikel nach Anspruch 2 oder 3, wobei die Oberfläche des Kontaktbereichs der Trennschicht eine Rauheit aufweist, die bei Messung mit einem auf 50fache Vergrößerung eingestellten konfokalen Lasermikroskop LEXT OLS3100 (Softwareversion 6.0) mit der hierin beschriebenen Methode bei einer Vergrößerung von 50x weniger als 0,12 $\mu$m, vorzugsweise weniger als 0,11 $\mu$m, stärker bevorzugt weniger als 0,1 $\mu$m und noch stärker bevorzugt weniger als 0,09 $\mu$m beträgt.

5. Depilierartikel nach einem der vorhergehenden Ansprüche, wobei die Depilierzusammensetzung bei Messung mit einem spannungsgesteuerten Amplitudensweep und einer Frequenz von 1 Hz sowie einer Temperatur von 25 °C eine Fließgrenze von 25 bis 2000 Pa, vorzugsweise von 40 bis 1000 Pa, stärker bevorzugt von 55 bis 500 Pa und noch stärker bevorzugt von 70 bis 250 Pa aufweist.

6. Depilierartikel nach einem der vorhergehenden Ansprüche, wobei die Depilierzusammensetzung wässrig ist, wobei vorzugsweise die Depilierzusammensetzung Wasser in einer Menge von mindestens 40 Gew.-%, vorzugsweise von 50 Gew.-% bis 98 Gew.-%, stärker bevorzugt von 60 Gew.-% bis 95 Gew.-% und noch stärker bevorzugt von 70 Gew.-% bis 90 Gew.-% der wässrigen Depilierzusammensetzung umfasst.

7. Depilierartikel nach einem der vorhergehenden Ansprüche, wobei der mindestens eine Teil des beschichteten Bereichs des Substrats durch eine Koronabehandlung, atmosphärische Plasmabehandlung, Flammenplasmabehandlung, chemische Plasmabehandlung oder Kombinationen davon behandelt wurde, wobei vorzugsweise zumindest ein Teil des beschichteten Bereichs durch Koronabehandlung behandelt wurde.

8. Depilierartikel nach einem der vorhergehenden Ansprüche, wobei das Substrat ein Polyethylen, vorzugsweise jedoch ein Niederdruckpolyethylen umfasst.

9. Depilierartikel nach einem der vorhergehenden Ansprüche, wobei die Depilierzusammensetzung auf dem Substrat in einer Menge pro Flächeneinheit des beschichteten Bereichs von 0,3 g/cm$^2$ bis 0,001 g/cm$^2$, vorzugsweise von 0,015 g/cm$^2$ bis 0,003 g/cm$^2$, stärker bevorzugt 0,08 g/cm$^2$ bis 0,005 g/cm$^2$ und noch stärker bevorzugt von 0,05 g/cm$^2$ bis 0,005 g/cm$^2$ angeordnet ist.

10. Depilierartikel nach einem der vorhergehenden Ansprüche, wobei das Substrat eine Dicke von 80 $\mu$m bis 12 $\mu$m, vorzugsweise von 50 $\mu$m bis 15 $\mu$m, stärker bevorzugt von 40 $\mu$m bis 16 $\mu$m und noch stärker bevorzugt von 30 $\mu$m bis 17 $\mu$m aufweist.

11. Depilierartikel nach einem der vorhergehenden Ansprüche, wobei die Depilierzusammensetzung ein Keratinreduktionsmittel, vorzugsweise Thioglycolsäure oder ein Thioglycolatsalz, stärker bevorzugt ein Thioglycolatsalz, noch stärker bevorzugt ein Thioglycolatsalz, umfassend ein zweiwertiges Kation, noch stärker bevorzugt Thioglycolatsalz, ausgewählt aus Natrium, Kalium, Magnesium, Calcium, Beryllium, Strontium, Zink, Monoethanolamin, Ammonium, Tetraalkylammonium, Imidazolium, Pyridinium, Phosphonium oder Glycerylthioglycolatsalze und noch stärker bevorzugt Calciumthioglycolat umfasst.

12. Depilierartikel nach einem der vorhergehenden Ansprüche, wobei die Depilierzusammensetzung einen Grundbestandteil, vorzugsweise in einem Konzentrationsbereich von 0,1 Gew.-% bis 10,0 Gew.-%, stärker bevorzugt von 0,5 Gew.-% bis 8 Gew.-% und noch stärker bevorzugt von 1 Gew.-% bis 5 Gew.-% der Depilierzusammensetzung umfasst.

13. Depilierartikel nach einem der vorhergehenden Ansprüche, wobei das Substrat wasserundurchlässig ist.

**14.** Verfahren zum Entfernen von Haaren von der Haut, das die folgenden Schritte umfasst:

(a) Auftragen eines Depilierartikels nach einem der vorstehenden Ansprüche auf eine Oberfläche der Haut, vorzugsweise auf menschliche Haut,
(b) Belassen des Depilierartikels in Kontakt mit der Haut für einen Zeitraum von mehr als 1 Minute, vorzugsweise 2 bis 10 Minuten, stärker bevorzugt 2 bis 8 Minuten,
(c) Entfernen des Depilierartikels von der Oberfläche der Haut und
(d) vorzugsweise Reiben, Kratzen, Spülen oder Abreiben der Oberfläche der Haut in dem Bereich, auf den der Depilierartikel aufgetragen wurde.

**15.** Depiliersatz, der Folgendes umfasst:

(a) einen Depilierartikel nach einem der Ansprüche 1 bis 13,
(b) wahlweise mindestens eines der Folgenden: eine Hautpflegevorbehandlungszusammensetzung, eine Hautpflegenachbehandlungszusammensetzung und/oder ein Werkzeug, um das Entfernen von Haaren und/oder der Depilierzusammensetzung nach Gebrauch zu unterstützen, und
(c) Verpackung für den Depiliersatz.


**Revendications**

**1.** Article dépilatoire comprenant :

i) un substrat comprenant une polyoléfine ; et
ii) une composition dépilatoire disposée sur ledit substrat, formant une région revêtue du substrat ;

dans lequel au moins une partie de la surface de la région revêtue du substrat a une énergie libre en surface moyenne mesurée en utilisant une technique de goutte sessile et un procédé de Fowkes tels que décrits ici allant de 31 mJ/m$^2$ à 72 mJ/m$^2$, de préférence de 32 mJ/m$^2$ à 55 mJ/m$^2$, plus préférablement de 33 mJ/m$^2$ à 46 mJ/m$^2$, même plus préférablement encore de 34 mJ/m$^2$ à 38 mJ/m$^2$ et encore plus préférablement de 35 mJ/m$^2$ à 37 mJ/m$^2$.

**2.** Article dépilatoire selon la revendication 1, dans lequel une couche protectrice détachable (3) est fixée de manière amovible à la composition dépilatoire de l'article dépilatoire, formant une région de mise en contact de la couche détachable ;
dans lequel, de préférence, la surface de la région de mise en contact de la couche détachable a une énergie libre en surface moyenne inférieure à l'énergie libre en surface moyenne de la région revêtue du substrat, plus préférablement la différence est d'au moins 2 mJ/m$^2$, encore plus préférablement la différence est d'au moins 4 mJ/m$^2$, même plus préférablement encore la différence est d'au moins 6 mJ/m$^2$, encore plus préférablement la différence est d'au moins 8 mJ/m$^2$ et même idéalement la différence est d'au moins 10 mJ/m$^2$.

**3.** Article dépilatoire selon l'une ou l'autre revendication précédente, dans lequel la surface de la région revêtue du substrat a une rugosité telle que mesurée en utilisant un microscope confocal à balayage laser à logiciel LEXT OLS3100 (Version 6.0) opérant à un agrandissement de x50 par le procédé décrit ici à un agrandissement de x50 d'au moins 0,08 $\mu$m, de préférence 0,09 $\mu$m, plus préférablement 0,1 $\mu$m, encore plus préférablement 0,11 $\mu$m et même plus préférablement encore 0,12 $\mu$m

**4.** Article dépilatoire selon la revendication 2 ou 3, dans lequel la surface de la région de mise en contact de la couche détachable a une rugosité, telle que mesurée en utilisant un microscope confocal à balayage laser à logiciel LEXT OLS3100 (Version 6.0) opérant à un agrandissement de x50 par le procédé décrit ici à un agrandissement de 50x, de moins de 0,12 $\mu$m, de préférence moins de 0,11 $\mu$m, plus préférablement moins de 0,1 $\mu$m et encore plus préférablement moins de 0,09 $\mu$m

**5.** Article dépilatoire selon l'une quelconque des revendications précédentes, dans lequel la composition dépilatoire a une limite de résistance élastique allant de 25 à 2000 Pa, de préférence de 40 à 1000 Pa, plus préférablement de 55 à 500 Pa et encore plus préférablement de 70 à 250 Pa lorsque l'on mesure par l'intermédiaire d'un balayage à amplitude contrôlée en contrainte à une fréquence de 1 Hz et une température de 25 °C.

**6.** Article dépilatoire selon l'une quelconque des revendications précédentes, dans lequel la composition dépilatoire est aqueuse, de préférence dans lequel la composition dépilatoire comprend de l'eau en une quantité d'au moins 40 %, de préférence de 50 % à 98 %, plus préférablement de 60 % à 95 % et encore plus préférablement de 70 % à 90 % en poids de la composition dépilatoire aqueuse.

7. Article dépilatoire selon l'une quelconque des revendications précédentes, dans lequel l'au moins une partie de la région revêtue du substrat a été traitée par traitement corona, par traitement au plasma atmosphérique, traitement au plasma à la flamme, traitement au plasma chimique ou leurs combinaisons, de préférence dans lequel au moins une partie de la région revêtue du substrat a été traitée par traitement corona.

8. Article dépilatoire selon l'une quelconque des revendications précédentes, dans lequel ledit substrat comprend un polyéthylène, de préférence encore du polyéthylène à haute densité.

9. Article dépilatoire selon l'une quelconque des revendications précédentes, dans lequel ladite composition dépilatoire est disposée sur ledit substrat en une quantité par surface unitaire de la région revêtue allant de 0,3 g/cm$^2$ à 0,001 g/cm$^2$, de préférence de 0,015 g/cm$^2$ à 0,003 g/cm$^2$, plus préférablement 0,08 g/cm$^2$ à 0,005 g/cm$^2$ et encore plus préférablement de 0,05 g/cm$^2$ à 0,005 g/cm$^2$.

10. Article dépilatoire selon l'une quelconque des revendications précédentes, dans lequel ledit substrat a une épaisseur allant de 80 $\mu$m à 12 $\mu$m, de préférence de 50 $\mu$m à 15 $\mu$m, plus préférablement de 40 $\mu$m à 16 $\mu$m, et encore plus préférablement de 30 $\mu$m à 17 $\mu$m.

11. Article dépilatoire selon l'une ou l'autre revendication précédente, dans lequel la composition dépilatoire comprend un agent réducteur de kératine, de préférence de l'acide thioglycolique ou un sel thioglycolate, plus préférablement un sel thioglycolate, encore plus préférablement un sel thioglycolate comprenant un cation divalent, même plus préférablement encore un sel thioglycolate choisi parmi les sels thioglycolate de sodium, potassium, magnésium, calcium, béryllium, strontium, zinc, monoéthanolamine, ammonium, tétralkylammonium, imidazolium, pyridinium, phosphonium ou glycéryle et encore plus préférablement le thioglycolate de calcium.

12. Article dépilatoire selon l'une quelconque des revendications précédentes, dans lequel la composition dépilatoire comprend une base, de préférence dans un intervalle de concentration allant de 0,1 % à 10,0 %, plus préférablement de 0,5 % à 8 % et encore plus préférablement de 1 % à 5 % en poids de la composition dépilatoire.

13. Article dépilatoire selon l'une quelconque des revendications précédentes, dans lequel le substrat est imperméable à l'eau.

14. Procédé d'élimination de poils de la peau, comprenant les étapes consistant à :

   (a) appliquer un article dépilatoire selon l'une quelconque des revendications précédentes sur une surface de peau, de préférence la peau humaine,
   (b) laisser ledit article dépilatoire en contact avec la peau pendant une période supérieure à 1 minute, de préférence 2 à 10 minutes, plus préférablement 2 à 8 minutes
   (c) retirer ledit article dépilatoire de la surface de la peau, et
   (d) de préférence frotter, racler, rincer ou essuyer la surface de la peau dans la zone sur laquelle l'article dépilatoire a été appliqué.

15. Trousse dépilatoire, comprenant :

   (a) un article dépilatoire selon l'une quelconque des revendications 1 à 13,
   (b) facultativement, au moins un élément parmi une composition de soin de la peau de prétraitement, une composition de soin de la peau de post-traitement et/ou un outil pour faciliter le retrait des poils et/ou de la composition dépilatoire après utilisation, et
   (c) un conditionnement pour ladite trousse dépilatoire.

# Fig.1.

# Fig.2.

# Fig.3.

**EP 2 559 417 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6192056 A
- US 2006002878 A
- JP 6135826 A
- JP 11012123 A
- JP 62230711 A
- JP 63073910 A

### Non-patent literature cited in the description

- **FOWKES, F.M.** ATTRACTIVE FORCES AT INTERFACES. *Industrial & Engineering Chemistry,* 1964, vol. 56 (12), 40-52
- **FOWKES, F.M.** Donor-Acceptor Interactions at Interfaces. *The Journal of Adhesion,* 1972, vol. 4 (2), 155-159